# EUROPÄISCHE PATENTSCHRIFT

(11) **EP 1 320 632 B1**
(45) Veröffentlichungstag und Bekanntmachung des Hinweises auf die Patenterteilung: **14.06.2006**
(21) Anmeldenummer: 01969203.7
(22) Anmeldetag: 02.08.2001
(51) Int. Cl.: C12Q 1/68

(54) **VERFAHREN ZUR ALTERSBESTIMMUNG VON INDIVIDUEN**
METHOD FOR DETERMINING THE AGE OF INDIVIDUALS
PROCEDE SERVANT A DETERMINER L'AGE D'INDIVIDUS

(30) Priorität: 02.08.2000 DE 10038733
(43) Veröffentlichungstag der Anmeldung: 25.06.2003
(73) Patentinhaber: Epigenomics AG, 10178 Berlin (DE)
(72) Erfinder: OLEK, Alexander, 10115 Berlin (DE); LEWIN, Jörn, 10787 Berlin (DE); ECKHARDT, Florian Dr., 10405 Berlin (DE)
(74) Vertreter: Schubert, Klemens
(86) Internationale Anmeldenummer: PCT/DE2001/002916
(87) Internationale Veröffentlichungsnummer: WO 2002/010445

(56) Entgegenhaltungen:
- WO-A-00/26401
- WO-A-98/54310
- CHOI E K ET AL: "ALTERATIONS OF C-FOS GENE METHYLATION IN THE PROCESSES OF AGING AND TUMORIGENESIS IN HUMAN LIVER" MUTATION RESEARCH, AMSTERDAM, NL, Bd. 354, 5. Juli 1996 (1996-07-05), Seiten 123-128, XP001026657 ISSN: 0027-5107
- AHUJA N ET AL: "AGING AND DNA METHYLATION IN COLORECTAL MUCOSA AND CANCER" CANCER RESEARCH, AMERICAN ASSOCIATION FOR CANCER RESEARCH, BALTIMORE, MD, US, Bd. 58, Nr. 23, 1. Dezember 1998 (1998-12-01), Seiten 5489-5494, XP001026172 ISSN: 0008-5472
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048

## Beschreibung

Die Erfindung betrifft ein Verfahren zur Alterbestimmung von Individuen.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußert sich das Alter eines Individuums in einem veränderten Methylierungsmuster einzelner Gene oder des Genoms.

Immer häufiger wird bei der Verfolgung von Straftaten am Tatort oder beim Opfer festgestelltes biologisches Material als Spurenmaterial sichergestellt, mittels DNA-Analyse untersucht und mit anderen DNA-Materialien verglichen.

In Anbetracht der weltweiten Forschung im Bereich der Genom-Analyse können inzwischen konkrete Aussagen über genetische Dispositionen mit inhaltlichem Informationswert getroffen werden. Die Speicherung und Nutzung der durch die DNA-Analyse gewonnenen Untersuchungsergebnisse in Datenbanken für erkennungsdienstliche Zwecke ist von herausragender Bedeutung.

Die Typisierung von biologischem Material gehört seit 1985 zu den wichtigsten Methoden bei der individuellen Identifizierung in der forensischen Medizin und bei kriminologischen Untersuchungen (Jeffreys A J, Wilson V, Thein S L. Hypervariable 'minisatellite' regions in human DNA. Nature. 1985 Mar 7-13;314(6006):67-73; .Benecke M. DNA typing in forensic medicine and in criminal investigations: a current survey. Naturwissenschaften 1997 May;84(5):181-8). Neben DNA Fingerprinting zur Personenidentifizierung gibt es auch die Möglichkeit, das Alter von männlichen Individuen anhand des Methylierungsmusters von Spermien festzustellen.

Geschlechts- und sequenzspezifische Methylierungsmuster von Säugetier-DNA etablieren sich während der Gametogenese. Es wird angenommen, dass sie entscheidend an genomischem Imprinting und an entwicklungsbedingter Genregulation beteiligt sind. Untersuchungen, die sich mit der Expression von an der DNA Methylierung beteiligten Enzymen befassen, zeigen, dass ein solches Enzym während der Spermatogenese auf der Stufe von mRNA, Protein und Enzymaktivität eindeutig entwicklungsbiologisch reguliert wird (Benoit G, Trasler JM. Developmental expression of DNA methyltransferase messenger ribonucleic acid, protein, and enzyme activity in the mouse testis. Biol Reprod. 1994 Jun:50(6):1312-9). Änderungen im 5-Methyldeoxycytidin Muster der DNA beeinflussen die Genexpression bestimmter Säugergene bezüglich Entwicklung, Differenzierung, Carcinogenese und des Alterns. Der Nachweis der DNA Methylierung in der Promotorregion, ein Vorgang, der normalerweise die Transkriptionsaktivität unterdrückt, ist ein wichtiges Untersuchungskriterium im Zusammenhang mit Veränderungen der molekularen Expression von altersbedingten Erkrankungen (Nagane Y, Utsugisawa K, Tohgi H. PCR amplification in bisulfite methylcytosine mapping in the GC-rich promoter region of amyloid precursor protein gene in autopsy human brain. Brain Res Brain Res Protoc. 2000 Apr;5(2):167-71.).

Der Methylierungszustand von Genen, welche CpG reiche Regionen (CpG Inseln) enthalten, wurde in menschlichen Spermien, fetalem und adultem Gewebe untersucht (Ghazi H, Gonzales FA, Jones PA. Methylation of CpG-islandcontaining genes in human sperm, fetal and adult tissues. Gene. 1992 May 15;114(2): 203-10). Es wurden Veränderungen der Methylierung während verschiedener Stadien der Entwicklung bei unterschiedlichen menschlichen Genen untersucht. Bei einem dieser Gene, welches für Insulin codiert, wurde nachgewiesen, dass es umfangreich in Spermien methyliert war, dass es unabhängig von der Expression in fetalem Gewebe weniger methyliert war und eine verstärkte Methylierung in adultem Gewebe vorlag. In jüngeren Untersuchungen, die sich mit dem speziellen Methylierungsmuster von aus Spermien isolierter DNA von Faktor VIII beschäftigten, konnte gezeigt werden, dass es nicht nur Unterschiede in der Mutationsfrequenz zwischen den CpG Stellen sondern auch zwischen zwei ethnischen Gruppen gibt. Die Ergebnisse verdeutlichen weiterhin, dass verschiedene CpG Stellen in ihrem Methylierungsmuster nicht nur innerhalb desselben Individuums variieren, sondern auch zwischen verschiedenen Individuen (Millar DS, Krawczak M, Cooper DN. Variation of site-specific methylation patterns in the factor VIII (F8C) gene in human sperm DNA. Hum Genet. 1998 Aug:103(2):228-33.)

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer.PCR-Amplifikation die epigenetische Information, welche die 5-Methylcytosine tragen, vollständig verloren.

Eine relativ neue und die mittlererweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z. B. Zechnigk, M. et al., Eur. J. Hum. Gen. 1997, 5, 94-98) nur in der Forschung angewendet. Immer aber werden kurze, spezifische Stücke eines bekannten Gens nach einer Bisulfit-Behandlung amplifziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO-A 9500669) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO-A 9928498).

Weitere Publikationen, die sich mit der Anwendung der Bisulfit-Technik zum Methylierungsnachweis bei einzelnen Genen befassen, sind: Xiong, Z. und Laird, P. W. (1997), Nucl. Acids Res. 25, 2532; Gonzalgo, M. L. und Jones, P. A. (1997), Nucl. Acids Res. 25, 2529; Grigg, S. und Clark, S. (1994), Bioassays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO-A 9746705 und WO-A 9515373.

Eine Übersicht über den Stand der Technik in der Oligomer Array Herstellung läßt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonukleotide bei vermindertem nichtspezifischem Hintergrundsignal entnehmen.

Ferner beschreiben N. Ahuja et al. ("Aging and DNA Methylation in Colorectal Muccosa and Cancer", Cancer Research, Bd. 58, 1998, 5489-5494) eine Studie, bei der Methylierungsanalysen von Darmkrebsproben durchgeführt wurden.

E. K. Choi et al. ("Alterations of c-fos gene methylation in the processes of aging and tumorigenesis in human liver", Mutation Research, Bd. 354, 1996, 123-128) beschreiben molekulare Mechanismen von Mutationen.

T. Rein et al. ("Identifying 5-methylcytosine and related modifications in DNA genomes", Bd. 26, 1998, 2255-2264) beschreiben eine Methylierungsanalyse durch genomische Sequenzierung bisulfitmodifizierter DNA.

Ein Verfahren zur Identifikation von Cytosin-Methylierungsmustern in genomischer DNA wird in der DE 199 05 082 beschrieben.

In der WO 97/46705 und in der WO 98/56952 werden Methylierungsnachweise beschrieben. Die WO 97/46705 beschreibt die Identifikation von DNA Methylierungsmustern in einer CpG enthaltenden Nukleinsäure und die WO 98/56952 ein Verfahren zur Diagnose von Krebs basierend auf Unterschieden in der Methylierung der DNA.

Aufgabe der Erfindung ist es, ein Verfahren bereitzustellen, das die Analyse des Alters von Individuen mittels molekularbiologischer Methoden erlaubt. Dazu sollen DNA Methylierungsmuster verwendet werden. Dabei ist vor allem das Problem zu lösen, dass diese speziellen Methylierungsmuster nicht von der ethnischen Zugehörigkeit und oder von z. B. Umwelteinflüssen abhängen dürfen, welche die Altersbestimmung andernfalls verfälschen.

Die vorliegende Erfindung beschreibt somit ein Verfahren zur Detektion des Methylierungszustandes genomischer DNA Proben mit dem Ziel, das Alter eines Individuums zu bestimmen.

Die Aufgabe wird durch ein Verfahren zur Bestimmung des Alters von Individuen gelöst, wobei man das DNA-Methylierungsmuster in einer DNA-Probe des Individuums analysiert und wobei man folgende Verfahrensschritte ausgeführt:
a) die in den Proben enthaltene DNA mit eine Bisulfit-lösung umgesetzten, wobei 5-Methylcytosin und Cytosin unterschiedlich reagieren und selektiv das Cytosin in eine Base mit von Cytosin abweichendem Basenpaarungsverhalten umgewandelt wird;
b) Teile der Nukleobasensequenz der DNA-Abschnitte bestimmt werden;
c) die erhaltenen Nukleobasensequenzen mit einer Datenbank abgeglichen werden, welche Sequenzen mit dem Alter von Individuen korreliert.

Ganz besonders bevorzugt ist es dabei, dass die DNA-Probe eine Spermaprobe ist.

Erfindungsgemäß bevorzugt ist ein Verfahren, wobei man
a) bestimmte CpG Dinukleotide in genomischer DNA auf ihren Methylierungsgrad hin analysiert und zwar getrennt für verschiedene Probanden verschiedenen Alters;
b) den Methylierungsgrad dieser CpG Dinukleotide mit dem Alter der Probanden korreliert und diese Informationen in einer Datenbank speichert;
c) das Alter eines Individuums aufgrund der Methylierungsanalyse der CpG Dinukleotide einer DNA-Probe des Individuums durch Abgleich der Analyseergebnisse mit der Datenbankinformation bestimmt.

Bevorzugt ist erfindungsgemäß ferner ein Verfahren, wobei man
a) die in den Proben enthaltene DNA chemisch derart behandelt, dass 5-Methylcytosin und Cytosin unterschiedlich reagieren und selektiv das Cytosin in eine Base mit von Cytosin abweichendem Basenpaarungsverhalten umgewandelt wird;
b) die Fragmente der derart behandelten DNA amplifiziert;
c) die Fragmente an einen Satz von Oligomeren hybridisiert;
d) die nicht hybridisierten Fragmente entfernt;
e) die hybridisierten Fragmente analysiert und das Ergebnis mit einer Datenbank abgleicht, welche die Hybridisierungsmuster mit dem Alter von Individuen korreliert.

Bevorzugt ist erfindungsgemäß ferner ein Verfahren, wobei man
a) die in den Proben enthaltene DNA chemisch derart behandelt, dass 5-Methylcytosin und Cytosin unterschiedlich reagieren und selektiv das Cytosin in eine Base mit von Cytosin abweichendem Basenpaarungsverhalten umgewandelt wird;
b) die Fragmente der derart behandelten DNA amplifiziert;
c) die Fragmente an einen Satz von Primeroligonukleotiden hybridisiert;
d) die Primer in einer sequenzspezifischen Reaktion verlängert;
e) die Verlängerungsprodukte analysiert und das Ergebnis mit einer Datenbank abgleicht, welche die Analyseergebnisse mit dem Alter von Individuen korreliert.

Ganz besonders bevorzugt ist beim erfindungsgemäßen Verfahren, dass man die Analyse mit Oligonukleotid-Arrays durchgeführt.

Ganz besonders bevorzugt ist beim erfindungsgemäßen Verfahren auch, dass man die Analyse mittels Massenspektrometrie durchgeführt.

Beschrieben wird also ein erfindungsgemäßes Verfahren zur Bestimmung des Alters von Individuen vorzugsweise anhand von Spermaproben.

Die Information zur Bestimmung des Alters von Individuen wird erhalten durch ein Verfahren zur Analyse von DNA-Methylierungsmustern in einer DNA-Probe, wobei die in den Proben enthaltene DNA mit einer Bisulfit-lösung umgesetzt, wobei 5-Methylcytosin und Cytosin unterschiedlich reagieren und selektiv das Cytosin in eine Base mit von Cytosin abweichendem Basenpaarungsverhalten umgewandelt wird. Die anschließende alkalische Hydrolyse führt dann zur Umwandlung von nicht methylierten Cytosin-Nukleobasen in Uracil. Anschließend werden Teile der Nukleobasensequenz der DNA-Abschnitte bestimmt und die erhaltenen Nukleobasensequenzen mit einer Datenbank abgeglichen, die die Sequenzen mit dem Alter von Individuen korreliert.

In einer besonders bevorzugten Ausführungsform des Verfahrens zur Bestimmung des Alters von Individuen werden Spermaproben untersucht, wobei die Altersinformation durch Analyse von DNA-Methylierungsmustern der in den Spermaproben enthaltenen Proben-DNA erhalten wird.

Für diese.Bestimmung des Alters von Individuen gibt es vorzugsweise folgende Ausführungsformen:

In einer bevorzugten Ausführungsform werden definierte CpG Dinukleotide in genomischer DNA auf ihren Methylierungsgrad hin untersucht und zwar getrennt für verschiedene Probanden verschiedenen Alters. Der Methylierungsgrad der CpG Dinukleotide wird mit dem Alter der Probanden korreliert und diese Informationen in einer Datenbank gespeichert. Das Alter eines Individuums wird aufgrund der Methylierungsanalyse der CpG Dinukleotide einer DNA-Probe des Individuums durch Abgleich der Analyseergebnisse mit der Datenbankinformation bestimmt.

In einer wiederum bevorzugten Ausführungsform wird die in den Proben enthaltene DNA mit einer Bisulfit-lösung umgesetzt, wobei 5-Methylcytosin und Cytosin unterschiedlich reagieren und selektiv das Cytosin in eine Base mit von Cytosin abweichendem Basenpaarungsverhalten umgewandelt wird. Dann werden Fragmente der vorbehandelten DNA unter Verwendung einer hitzebeständigen Polymerase und mindestens einem Primer bevorzugt mit der Polymerasereaktion (PCR) amplifiziert. Es werden verschiedene definierte Amplifikationen in einem Reaktionsgefäß durchgeführt. Dies ist vorzugsweise eine sogenannte Multiplex PCR, in der verschiedene Primer jeweils definierte Fragmente erzeugen. In einer weiteren Variante des Verfahrens amplifizieren Primer gezielt und reproduzierbar jeweils mehrere Fragmente. In einer besonders bevorzugten Variante des Verfahrens findet die Amplifikation durch die Verlängerung von Primern statt, die an eine Festphase gebunden sind. Eine Multiplex-PCR im weiteren Sinne kann dadurch ausgeführt werden, dass unterschiedliche Primer an verschiedenen, definierten Orten einer Festphase gebunden sind. Die Festphase ist in der Regel eben, wobei die unterschiedlichen Oligonukleotidsequenzen in Form eines rechtwinkligen oder hexagonalen Gitters angeordnet sind. Das hat zur Folge, dass auch die unterschiedlichen Amplifikate auf der Festphase in Form eines rechtwinkligen oder hexagonalen Gitters angeordnet sind. Wie bereits oben beschrieben, werden in diesem Fall mehrere Amplifikate direkt auf der Festphase erzeugt. Die Festphasenoberfläche besteht vorzugsweise aus Silizium, Glas, Polystyrol, Aluminium, Stahl, Eisen, Kupfer, Nickel, Silber oder Gold. Die Fragmente der amplifizierten genomischen DNA werden an einen Satz von Oligomeren (Primern) unter Duplex-Ausbildung hybridisiert. Die nicht hybridisierten Fragmente werden anschließend entfernt. Abschließend werden die hybridisierten Fragmente analysiert und das Ergebnis mit einer Datenbank abgeglichen, welche die Hybridisierungsmuster mit dem Alter von Individuen korreliert.

In einer besonders bevorzugten Ausführungsform wird die in den Proben enthaltene DNA mit einer Bisulfit-lösung umgesetzt, wobei 5-Methylcytosin und Cytosin unterschiedlich reagieren und selektiv das Cytosin in eine Base mit von Cytosin abweichendem Basenpaarungsverhalten umgewandelt wird. Dann werden Fragmente der vorbehandelten DNA unter Verwendung einer hitzebeständigen Polymerase und mindestens einem Primer bevorzugt mit der Polymerasereaktion (PCR) amplifiziert. Es werden verschiedene definierte Amplifikationen in einem Reaktionsgefäß durchgeführt. Dies ist vorzugsweise eine sogenannte Multiplex PCR, in der verschiedene Primer jeweils definierte Fragmente erzeugen. In einer weiteren Variante des Verfahrens amplifizieren Primer gezielt und reproduzierbar jeweils mehrere Fragmente. In einer besonders bevorzugten Variante des Verfahrens findet die Amplifikation durch die Verlängerung von Primern statt, die an eine Festphase gebunden sind. Eine Multiplex-PCR im weiteren Sinne kann dadurch ausgeführt werden, dass unterschiedliche Primer an verschiedenen, definierten Orten einer Festphase gebunden sind. Die Festphase ist in der Regel eben, wobei die unterschiedlichen Oligonukleotidsequenzen in Form eines rechtwinkligen oder hexagonalen Gitters angeordnet sind. Das hat zur Folge, dass auch die unterschiedlichen Amplifikate auf der Festphase in Form eines rechtwinkligen oder hexagonalen Gitters angeordnet sind. Wie bereits oben beschrieben, werden in diesem Fall mehrere Amplifikate direkt auf der Festphase erzeugt. Die Festphasenoberfläche besteht vorzugsweise aus Silizium, Glas, Polystyrol, Aluminium, Stahl, Eisen, Kupfer, Nickel, Silber oder Gold. Die Fragmente werden anschließend an einen Satz von Primeroligonukleotiden hybridisiert und die Primer in einer sequenzspezifischen Reaktion mit einer hitzebeständigen Polymerase verlängert. Bevorzugt trägt dabei zumindest ein Nukleotid eine nachweisbare Markierung. Die Art der Verlängerung hängt dabei vom Methylierungsstatus des jeweiligen Cytosins in der genomischen DNA-Probe ab. Abschließend werden die Verlängerungsprodukte analysiert und das Ergebnis mit einer Datenbank abgeglichen, welche die Hybridisierungsmuster mit dem Alter von Individuen korreliert.

Die Analyse der DNA-Methylierungsmuster der vorstehenden Ausführungsformen wird besonders bevorzugt mittels Massenspektrometrie durchgeführt.

Die folgenden Beispiele erläutern die Erfindung:

### Beispiel 1:

### Beschreibung der PCR

Die Einzelgen-PCR-Reaktion wurde mittels eines Thermocyclers (Epperdorf GmbH) unter Verwendung von 10 ng Bisulfit-behandelter DNA, 12,5 pmol jedes Primers, 1mM jeder dNTP, 1,5 mM MgCl₂ und 1 U HotstartTaq (Qiagen AG) durchgeführt. Die anderen Bedingungen entsprachen denen, die der Taq-Polymerase-Hersteller empfiehlt. Einzelgene wurden mittels PCR amplifiziert, indem ein erster Denaturierungsschritt für 20 min. bei 95 °C, gefolgt von 45 Zyklen (60 sec. bei 95 °C, 45 sec. bei 55 °C, 75 sec. bei 72 °C) und eine nachfolgende abschließende Elongation von 10 min. bei 72 °C durchgeführt wurden.

Beispiel 2: Die folgenden Beispiele beziehen sich auf ein Fragment von Exon 40 des Gens FVIII, in welchem spezifische CG-Positionen auf Methylierung zu analysieren waren.

Im ersten Schritt ist eine genomische Sequenz unter Verwendung von Bisulfit (Hydrogensulfit, Disulfit) so behandelt worden, dass alle an der 5-Position der Base nicht methylierten Cytosine in solcher Art modifiziert werden, dass eine andere Base in Bezug auf das Basenpaarungsverhalten substituiert wird, während die in der 5-Position methylierten Cytosine unverändert bleiben.

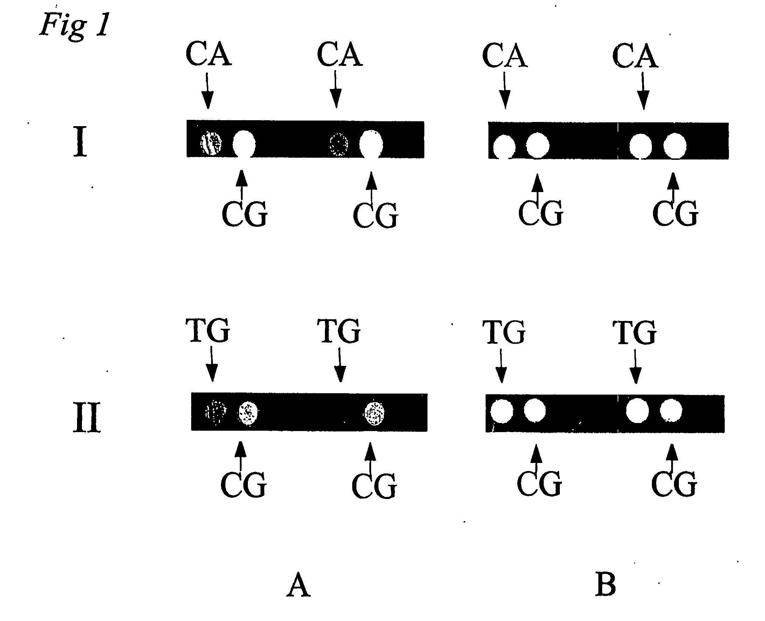

Wenn für die Reaktion Bisulfit-Lösung verwendet wird, findet an den nicht-methylierten Cytosin-Basen eine Addition statt. Ferner muss ein Denaturierungs-Reagenz oder Lösemittel sowie ein Radikalfänger anwesend sein. Eine nachfolgende alkalische Hydrolyse bewirkt dann die Umsetzung der nicht-methylierten Cytosin-Nukleobasen zu Uracil. Die chemisch umgewandelte DNA wird dann zur Detektion methylierter Cytosine verwendet. Im zweiten Verfahrensschritt wird die behandelte DNA-Probe mit Wasser oder einer wässrigen Lösung verdünnt. Bevorzugter Weise wird die DNA nachfolgend desulfoniert.
Im dritten Schritt des Verfahren wird die DNA-Probe in einer Polymerasekettenreaktion amplifiziert, bevorzugter Weise unter Verwendung einer hitzebeständigen DNA-Polymerase, wie in Beispiel 1 beschrieben. Im vorliegenden Fall werden Cytosine von Exon 11 des Gens FVIII analysiert. Zu diesem Zweck wird ein definiertes Fragment mit einer Länge von 561 bp mit den spezifischen PrimerOligonukleotiden AGGGAGTTTTTTTTAGGGAATAGAGGGA und TAATCCCAAAACCTCTCCACTACAACAA amplifiziert.
Das Amplifikat dient als Probe, die an Oligonukleotide hybridisiert, welche vorher an eine Feststoffphase gebunden wurden, und bildet eine Duplex-Struktur aus, zum Beispiel TTCCACTTAATCGCTCCC (das CG dieses Oligonukleotids wird in Fig. 1, I gezeigt) oder AGAGTTTTCGTAGTTTTT (das CG dieses Oligonukleotids wird in Fig. 1, II gezeigt), wobei sich das zu detektierende Cytosin an Position 30 beziehungsweise an Position 500 des Amplifikats befindet. Die Detektion des Hybridisierungs-Produkts basiert auf Cy5 fluoreszierend markierten Primer-Oligonukleotide, die für die Amplifizierung verwendet worden sind. Eine Hybridisierungs-Reaktion der amplifizierten DNA mit dem Oligonukleotid findet nur dann statt, wenn ein methyliertes Cytosin an dieser Stelle in der Bisulfit-behandelten DNA vorgelegen hat, wie in Figur 1 zeigt wird. Folglich wird der Methylierungszustand des spezifischen, zu analysierenden Cytosins aus dem Hybridisierungsprodukt abgeleitet.

Um den nicht methylierten Zustand zu analysieren, werden die Oligonukleotide TTCCACTTAATCACTCCC (das CA dieses Nukleotids wird in Fig. 1, I gezeigt) oder AGAGTTTTTGTAGTTTTT (das TG dieses Oligonukleotids wird in Fig. 1, II gezeigt) verwendet. An den zu analysierenden Positionen umfassen diese Oligonukleotide eine Thymin-Base an Stelle eines Cytosins. Deshalb findet die Hybridisierungsreaktion nur statt, wenn ein unmethyliertes Cytosin an der zu analysierenden Position vorlag, wie in Figur 1 gezeigt wird. Für zwei verschiedene Oligonukleotide wird gezeigt, dass die zu analysierenden CpG-Positionen einen unterschiedlichen Methylierungsgrad für einen 41 Jahre alten Probanden (Fig. 1, A) verglichen mit einem 23 Jahre alten Probanden (Fig. 1, B) zeigen. Die Signalintensitäten beider Oligonukleotide für den methylierten Zustand, dargestellt durch die CG enthaltenden Oligonukleotide des 41 Jahre alten Probanden (Fig. 1, A) sind höher als die Intensitäten, des den nicht methylierten Zustand repräsentierenden, CA enthaltenden Oligonukleotids dieses Patienten. Im Gegensatz dazu sind die Signalintensitäten der den methylierten Zustand repräsentierenden Oligonukleotide und die der den methylierten Zustand repräsentierenden Oligonukleotide für den 23 Jahre alten Probanden nahezu gleich (Fig. 1, B).

### Beschreibung der Figur 1

Figur 1 zeigt die Hybridisierung von fluoreszierend markierten Amplifikaten an oberflächengebundene Oligonukleotide für verschiedene Oligonukleotide (wie in Figur 1 in zweifacher Wiederholung für jedes Oligonukleotid gezeigt). Wobei die Oligonukleotid-Proben A vom 41 Jahre alten Probanden und Probe B vom 23 Jahre alten Probanden stammen. Fluoreszenz an einem Spot, gekennzeichnet durch einen Pfeil, weist auf Hybridisierung des Amplifikats am Oligonukleotid hin. Hybridisierung an ein CG-enthaltendes Oligonukleotid kennzeichnet Methylierung an der analysierten Cytosin-Position, Hybridisierung an ein CA beziehungsweise TG-enthaltendes Oligonukleotid kennzeichnet keine Methylierung an der zu analysierenden Cytosin-Position.

## Patentansprüche

1. Verfahren zur Bestimmung des Alters von Individuen, **dadurch gekennzeichnet, dass** man das DNA-Methylierungsmuster in einer DNA-Probe des Individuums analysiert und dass man folgende Verfahrensschritte ausgeführt:
a) die in den Proben enthaltene DNA mit einer Bisulfit-Lösung umgesetzt wird, wobei 5-Methylcytosin und Cytosin unterschiedlich reagieren und selektiv das Cytosin in eine Base mit von Cytosin abweichendem Basenpaarungsverhalten umgewandelt wird;
b) Teile der Nukleobasensequenz der DNA-Abschnitte bestimmt werden;
c) die erhaltenen Nukleobasensequenzen mit einer Datenbank abgeglichen werden, welche Sequenzen mit dem Alter von Individuen korreliert.

2. Verfahren zur Bestimmung des Alters von Individuen gemäß Anspruch 1, **dadurch gekennzeichnet, dass** die DNA-Probe eine Spermaprobe ist.

3. Verfahren zur Bestimmung des Alters von Individuen nach einem der Ansprüche 1 oder 2, **dadurch gekennzeichnet, dass** man
a) CpG Dinukleotide in genomischer DNA auf ihren Methylierungsgrad hin analysiert und zwar getrennt für verschiedene Probanden verschiedenen Alters;
b) den Methylierungsgrad dieser CpG Dinukleotide mit dem Alter der Probanden korreliert und diese Informationen in einer Datenbank speichert;
c) das Alter eines Individuums aufgrund der Methylierungsanalyse der CpG Dinukleotide einer DNA-Probe des Individuums durch Abgleich der Analyseergebnisse mit der Datenbankinformation bestimmt.

4. Verfahren zur Bestimmung des Alters von Individuen anhand von DNA-Proben nach einem der voranstehenden Ansprüche, **dadurch gekennzeichnet, dass** man
a) die in den Proben enthaltene DNA mit einer Bisulfit-Lösung umgesetzt wird, wobei 5-Methylcytosin und Cytosin unterschiedlich reagieren und selektiv das Cytosin in eine Base mit von Cytosin abweichendem Basenpaarungsverhalten umgewandelt wird;
b) die Fragmente der derart behandelten DNA amplifiziert;
c) die Fragmente an einen Satz von Oligomeren hybridisiert;
d) die nicht hybridisierten Fragmente entfernt;
e) die hybridisierten Fragmente analysiert und das Ergebnis mit einer Datenbank abgleicht, welche die Hybridisierungsmuster mit dem Alter von Individuen korreliert.

5. Verfahren zur Bestimmung des Alters von Individuen anhand von DNA-Proben nach einem der Ansprüche 1 bis 3, **dadurch gekennzeichnet, dass** man
a) die in den Proben enthaltene DNA mit einer Bisulfit-Lösung umgesetzt wird, wobei 5-Methylcytosin und Cytosin unterschiedlich reagieren und selektiv das Cytosin in eine Base mit von Cytosin abweichenden Basenpaarungsverhalten umgewandelt wird;
b) die Fragmente der derart behandelten DNA amplifiziert;
c) die Fragmente an einen Satz von Primeroligonukleotiden hybridisiert;
d) die Primer sequenzspezifisch verlängert;
e) die Verlängerungsprodukte analysiert und das Ergebnis mit einer Datenbank abgleicht, welche die Analyseergebnisse mit dem Alter von Individuen korreliert.

6. Verfahren nach einem der voranstehenden Ansprüche, **dadurch gekennzeichnet, dass** man die Analyse mit Oligonukleotid-Arrays durchgeführt.

7. Verfahren nach einem der Ansprüche 1 bis 6, **dadurch gekennzeichnet, dass** man die Analyse mittels Massenspektrometrie durchgeführt.

## Claims

1. A method for the determination of the age of individuals, hereby **characterized in that** the DNA methylation patterns are analyzed in a DNA sample of the individual, and that the following method steps are conducted:
a) the DNA contained in the samples is converted with a bisulfite solution, whereby 5-methylcytosine and cytosine react differently and cytosine is selectively converted into a base with a base-pairing behaviour that is different from that of cytosine;
b) portions of the base sequence of the DNA segments are determined;
c) the obtained nucleotide base sequences are compared with a database, which correlates sequences with the age of individuals.

2. The method for the determination of the age of individuals according to claim 1, further **characterized in that** the DNA sample is a sperm sample.

3. The method for the determination of the age of individuals according to claim 1 or 2, further **characterized in that**
a) CpG dinucleotides in genomic DNA are analyzed for their degree of methylation and this is done separately for different subjects of different age;
b) the degree of methylation of these CpG dinucleotides is correlated with the age of the subject and this information is stored in a database;
c) the age of an individual is determined on the basis of the methylation analysis of the CpG dinucleotides of a DNA sample of the individual by comparing the analytical results with the database information.

4. The method for the determination of the age of individuals on the basis of DNA samples according to one of the preceding claims, further **characterized in that**
a) the DNA contained in the samples is converted with a bisulfite solution, whereby 5-methylcytosine and cytosine react differently and cytosine is selectively converted into a base with a base-pairing behaviour that is different from that of cytosine;
b) the fragments of the DNA treated in this way are amplified;
c) the fragments hybridize to a set of oligomers;
d) the unhybridized fragments are removed;
e) the hybridized fragments are analyzed and the result is compared with a database, which correlates the hybridization pattern with the age of individuals.

5. The method for the determination of the age of individuals on the basis of DNA samples according to one of claims 1 to 3, further **characterized in that**
a) the DNA contained in the samples is converted with a bisulfite solution, whereby 5-methylcytosine and cytosine react differently and cytosine is selectively converted into a base with a base-pairing behaviour that is different from that of cytosine;
b) the fragments of the DNA treated in this way are amplified;
c) the fragments are hybridized to a set of primer oligonucleotides;
d) the primers are sequence-specific extended;
e) the extension products are analyzed and the result is compared with a database, which correlates the analytical results with the age of individuals.

6. The method according to one of the preceding claims, further **characterized in that** the analysis is conducted with oligonucleotide arrays.

7. The method according to one of claims 1 to 6, further **characterized in that** the analysis is conducted by means of mass spectrometry.

## Revendications

1. Procédé de détermination de l'âge d'individus, **caractérisé en ce que** l'on analyse le motif de méthylation de l'ADN dans un échantillon d'ADN de l'individu et **en ce que** l'on réalise les étapes de procédé suivantes:
a) on fait réagir l'ADN contenu dans les échantillons avec une solution de bisulfite, réaction au cours de laquelle la 5-méthylcytosine et la cytosine réagissent différemment et la cytosine est transformée sélectivement en une base ayant un comportement d'appariement de bases qui diffère de celui de la cytosine;
b)on détermine des parties de la séquence de bases nucléiques des segments d'ADN; et
c) on compare les séquences de bases nucléiques obtenues avec une banque de données qui met les séquences en corrélation avec l'âge des individus.

2. Procédé de détermination de l'âge d'individus selon la revendication 1, **caractérisé en ce que** l'échantillon d'ADN est un échantillon de sperme.

3. Procédé de détermination de l'âge d'individus selon l'une quelconque des revendications 1 ou 2, **caractérisé en ce que**:
a) on analyse les dinucléotides CpG d'ADN génomique pour déterminer leur degré de méthylation, cela se faisant séparément pour différents sujets expérimentaux d'âges variables;
b) on met en corrélation le degré de méthylation de ces dinucléotides CpG avec l'âge des sujets expérimentaux et on enregistre ces informations dans une banque de données;
c) on détermine l'âge d'un individu sur la base de l'analyse de méthylation des dinucléotides CpG d'un échantillon d'ADN de l'individu par comparaison des résultats d'analyse avec les informations de la banque de données.

4. Procédé de détermination de l'âge d'individus à l'aide d'échantillons d'ADN selon l'une quelconque des revendications précédentes, **caractérisé en ce que**:
a) on fait réagir l'ADN contenu dans les échantillons avec une solution de bisulfite, réaction au cours de laquelle la 5-méthylcytosine et la cytosine réagissent différemment et la cytosine est transformée sélectivement en une base ayant un comportement d'appariement de bases qui diffère de celui de la cytosine;
b) on amplifie les fragments de l'ADN ainsi traité;
c) on effectue l'hybridation des fragments avec un jeu d'oligomères;
d) on élimine les fragments non hybridés;
e) on analyse les fragments hybridés et on compare le résultat avec une banque de données qui met les motifs d'hybridation en corrélation avec l'âge des individus.

5. Procédé de détermination de l'âge d'individus à l'aide d'échantillons d'ADN selon l'une quelconque des revendications 1 à 3, **caractérisé en ce que**:
a) on fait réagir l'ADN contenu dans les échantillons avec une solution de bisulfite, réaction au cours de laquelle la 5-méthylcytosine et la cytosine réagissent différemment et la cytosine est transformée sélectivement en une base ayant un comportement d'appariement de bases qui diffère de celui de la cytosine;
b) on amplifie les fragments de l'ADN ainsi traité;
c) on effectue l'hybridation des fragments avec un jeu d'oligonucléotides d'amorçage;
d) on effectue l'élongation des amorces de manière spécifique aux séquences;
e) on analyse les produits d'élongation et on compare le résultat avec une banque de données qui met les résultats d'analyse en corrélation avec l'âge des individus.

6. Procédé selon l'une quelconque des revendications précédentes, **caractérisé en ce que** l'on effectue l'analyse avec des réseaux d'oligonucléotides.

7. Procédé selon l'une quelconque des revendications 1 à 6, **caractérisé en ce que** l'on effectue l'analyse par spectrométrie de masse.
